# EUROPEAN PATENT APPLICATION

(11) **EP 4 822 655 A1**
(43) Date of publication of application: **14.10.2026**
(21) Application number: 26170194.0
(22) Date of filing: 01.04.2026
(51) Int. Cl.: G06V 20/10, G01N 15/1433, G06V 20/70, E21B 49/00, G01N 33/24

(54) **AUTOMATED RIG SITE LITHOLOGY DATA EXTRACTION**

(30) Priority: 07.04.2025 EP 25305504; 14.05.2025 US 202519207562
(71) Applicant: Services Pétroliers Schlumberger, 75007 Paris (FR); Schlumberger Technology B.V., 2514 JG The Hague (NL)
(72) Inventor: WANTZ, François, 92140 Clamart (FR); SU, Laura, 92140 Clamart (FR); FRANCOIS, Matthias, 92140 Clamart (FR); BARNECHE, Anne-Maëlle, 92140 Clamart (FR)
(74) Representative: Schlumberger Intellectual Property Department

(57) **Abstract**

A computerized method for evaluating a drill cuttings lithology includes acquiring a digital image of drill cuttings and segmenting the acquired image at a rig site to demarcate individual particles in image. Features are extracted from selected ones of the demarcated particles in the segmented image and the demarcated particles are further assigned to at least one group based on the extracted features at the rig site. A rig site user is prompted to input measurement data, a label, or a description of the at least one group. The segmented image, the extracted features, the assigned grouping, and the user input are electronically transferred to a cloud computing resource which performs a lithological classification of selected ones of the demarcated particles based on the extracted features and the user input and generates consolidated summary results of the lithological classification.

## Description

### CROSS REFERENCE TO RELATED APPLICATIONS

### BACKGROUND

In subterranean drilling operations, such as for oil and gas exploration and production, formation cuttings are generated by the drilling activity. These cuttings have long been evaluated and characterized to better understand the properties and structure of subsurface rock. In recent years, there has been a desire to perform such evaluation and characterization automatically (or semi-automatically) to reduce human cost and shorten the turnaround time of the interpretation. However, implementing such automation is far from routine.

Automatic evaluation and characterization of digital drill cuttings images is particularly challenging since the individual cuttings include a wide variety of sizes, shapes, textures, and colors. During a single drilling operation, a large number of drill cuttings images of may be acquired. Moreover, each image includes numerous cuttings particles jumbled together on a tray with individual particles sometimes touching and partially overlapping one another. Extensive electronic memory and computer processing power is required to extract the particle features necessary for subsequent AI-based processing. Managing the processing power requirements and providing reliable and timely results for each of many drilling operations around the globe is a highly challenging problem. There is a need in the industry for improved automated methods for evaluating and classifying drill cuttings images.

### BRIEF DESCRIPTION OF THE DRAWINGS

For a more complete understanding of the disclosed subject matter, and advantages thereof, reference is now made to the following descriptions taken in conjunction with the accompanying drawings, in which:
FIG. 1 depicts an example drilling rig including an example system for evaluating cuttings particles.
FIG. 2 depicts a flow chart of one example method for generating a cuttings description during a drilling operation.
FIGS. 3A and 3B (collectively FIG. 3) depict a flow chart of another example method for generating a cuttings description during a drilling operation.
FIG. 4 depicts an example digital image of cuttings particles including first and second groups of cuttings particles.

### DETAILED DESCRIPTION

Computerized methods and systems for evaluating a drill cuttings lithology are disclosed. One example method embodiment comprises acquiring a digital image of drill cuttings at a rig site and segmenting the acquired digital image at the rig site to demarcate individual particles in the digital image. Features are extracted from selected ones of the demarcated particles in the segmented image at the rig site. The demarcated particles are further assigned to at least one group at the rig site based on the extracted features A rig site user is prompted to input measurement data, a label, or a description of the at least one group. The segmented image, the extracted features, the assigned grouping, and the user input are electronically transferred to a cloud computing resource which performs a lithological classification of selected ones of the demarcated particles based on the extracted features and the user input and generates consolidated summary results of the lithological classification.

As known to those of ordinary skill, drilling a subterranean borehole (e.g., in oil and gas exploration and/or production operations) generates a large volume of rock cuttings that are available at the rig site. In current drilling operations, these cuttings are commonly evaluated by geologists, petrophysicists, and/or other experts at the rig site, for example, to evaluate local lithology and the oil bearing potential of the well. The cuttings description and evaluation are generally manual and repetitive tasks performed at the rig site. A commonly implemented workflow requires rig personnel to identify each lithology type by performing chemical and/or physical measurements on cuttings samples. Upon identifying the lithology types in the cuttings sample, the operator describes the rock characteristics (color, hardness, grain size, etc.), for example, on paper.

In more recent times, there has been a strong desire to implement automated evaluation of the cuttings (e.g., via using artificial intelligence and neural networks to evaluate digital images of the cuttings). One motivating factor in implementing automated methods is to remove costly experts from the rig site. While such automated processing and evaluation is promising, there are also a number of difficulties as described above in the background section of the disclosure. The disclosed embodiments advantageously distribute the required computer processing to both rig site and cloud processors. By distributing some of the heavy processing to the rig site, the disclosed embodiments tend to reduce accumulation of data at a single remote location (the cloud) and thereby further tends to reduce lag time and promote efficient and reliable service (in terms of service availability). As described in more detail below, the disclosed embodiments may also accommodate (and even prompt) user input, which tends to improve the overall accuracy of the lithological classification.

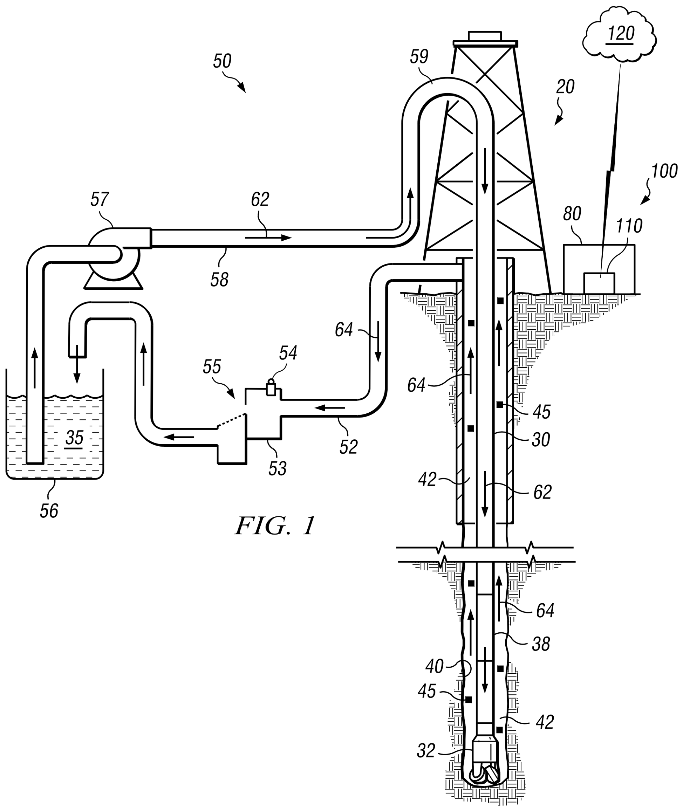

FIG. 1 depicts an example drilling rig 20 including a system 100 for evaluating cuttings particles that are removed from circulating drilling fluid on the rig. The drilling rig 20 may be positioned over a subterranean formation (not shown). The rig 20 may include, for example, a derrick and a hoisting apparatus (also not shown) for raising and lowering a drill string 30, which, as shown, extends into wellbore 40 and includes, for example, a drill bit 32 and one or more downhole measurement tools 38 (e.g., a logging while drilling tool or a measurement while drilling tool) in a bottom hole assembly (BHA) above the bit 32. Suitable drilling systems, for example, including drilling, steering, logging, and other downhole tools are well known in the art.

While FIG. 1 depicts a land rig 20, it will be appreciated that the disclosed embodiments are equally well suited for land rigs or offshore rigs. As is known to those of ordinary skill, offshore rigs commonly include a platform deployed atop a riser that extends from the sea floor to the surface. The drill string extends downward from the platform, through the riser, and into the wellbore through a blowout preventer (BOP) located on the sea floor. The disclosed embodiments are not limited in these regards.

Drilling rig 20 further includes a surface system 50 for controlling the flow of drilling fluid used on the rig (e.g., used in drilling the wellbore 40). In the example rig depicted, drilling fluid 35 is pumped downhole (as depicted at 62), for example, via a conventional mud pump 57. The drilling fluid 35 may be pumped, for example, through a standpipe 58 and mud hose 59 in route to the drill string 30. The drilling fluid 35 typically emerges from the drill string 30 at or near the drill bit 32 and creates an upward flow 64 of mud through the wellbore annulus 42 (the annular space between the drill string and the wellbore wall). The drilling fluid 35 then flows through a return conduit 52 to a mud pit system 56 where may be recirculated. It will be appreciated that the terms drilling fluid and mud are used synonymously herein.

The circulating drilling fluid 35 is intended to perform many functions during a drilling operation, one of which is to carrying drill cuttings 45 to the surface (in upward flow 64). The drill cuttings 45 are commonly removed from the returning mud via a shale shaker 55 (or other similar solids control equipment) in the return conduit (e.g., immediately upstream of the mud pits 56). Formation gases that are released during drilling may also be carried to the surface in the circulating drilling fluid. These gasses are commonly removed from the fluid, for example, via a degasser or gas trap 54 located in or near a header tank 53 that is immediately upstream of the shale shaker 55 in the example depiction. The drill cuttings 45 may be evaluated to characterize the subterranean formation and/or estimate various properties thereof as described in more detail below.

The rig 20 may include a system 100 configured to take and evaluate digital images of the drill cuttings as described in more detail below. The system 100 advantageously employs an embedded computing unit 110 deployed at the rig site (e.g., in an onsite laboratory 80). As depicted, the embedded computing unit 110 may be configured to be in electronic communication with cloud computing resources 120. A system workflow executed in the embedded computing unit 110 may be configured to combine artificial intelligence (AI) processing on digital images of cuttings particles with measured cuttings properties input by rig personnel. Merging the user inputs, e.g., as labels, with artificial intelligence data output by the computing unit 110, a smart workflow may precompute image data and feature extraction and thereby reduce computational demand on the cloud resources 120.

It will be appreciated that to perform the functionality described herein, the system 100 (including embedded unit 110 and cloud resources 120) may include computer hardware and software configured to automatically or semi-automatically evaluate the cuttings images. To perform these functions, the hardware may include one or more processors (e.g., microprocessors) which may be connected to one or more data storage devices (e.g., hard drives or solid state memory). As is known to those of ordinary skill, the processors may be further connected to a network interface e.g., to receive the images from a networked camera system (not shown) or another computer system as well as to enable communication with various surface and/or downhole sensors, gauges, and so forth as well as to communicate with actuators and other surface and/or downhole equipment. Moreover, a network interface may provide for communication between the embedded unit 110 and the cloud resources 120. It will, of course, be understood that the disclosed embodiments are not limited the use of or the configuration of any particular network configuration, computer hardware, and/or software.

With continued reference to FIG. 1, the rig 20 (e.g., the laboratory 80) may be configured to equip the acquisition of digital images of selected cuttings particles as well as the measurement of numerous physical properties of the cuttings particles and may therefore include various measurement tools and instrumentation. For example, the rig may include a calibrated image acquisition system including a digital camera, a lens, a light source, a cuttings sample tray, and a computer system configured to take and store the digital images. Moreover, the rig 20 may include a hardness or microhardness tool configured to measure a hardness value of individual cuttings particles as well as a porosimeter configured to measure porosity values of individual cuttings particles or groups of similar cuttings particles. The rig 20 may further include an ultraviolet (UV) florescence measurement apparatus as well as an imaging apparatus for measuring and sorting the grain size of the cuttings particles. The rig 20 may still further include other instrumentation (such as the SLB PetroFecta^{®}) configured, for example, to make x-ray fluorescence (XRF) elemental analysis measurements, diffuse reflectance infrared Fourier transform spectroscopy (DRIFTS) mineralogy measurements, and to take high resolution visible and ultraviolet (UV) digital images of the cuttings particles. These measurements and/or images may be evaluated by trained rig personnel to determine the lithology of the cuttings particles as well as the particle size and grain size. Moreover, the rig 20 may further include a chemical laboratory suitable for performing an acid test in which cuttings particles are exposed to a strong acid such as hydrochloric acid or sulfuric acid.

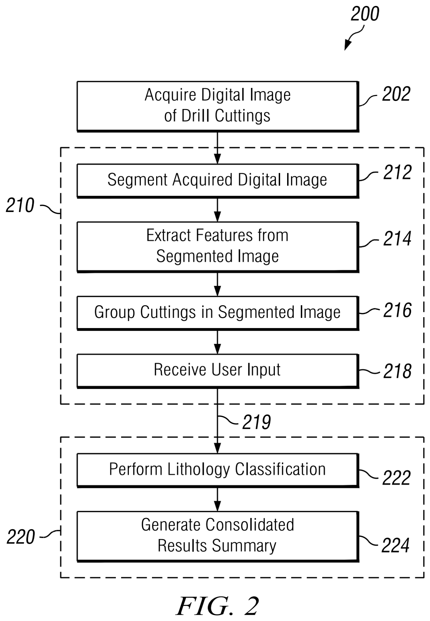

Turning now to FIG. 2, a flow chart of one example method 200 for generating a cuttings description during a drilling operation is depicted. Method 200 includes obtaining or taking a digital image of drill cuttings particles at 202. The cuttings particles may be generated, for example, while drilling a wellbore and may be transported to the surface in circulating drilling fluid as described above with respect to FIG. 1. The obtained digital images may be processed at the rig (as shown collectively at 210) to group the individual particles into one or more groups based on particle features and to prompt rig personnel to input measured physical property data and/or a description or classification for one of more of the particle groups (e.g., each of the particle groups). The digital images, the grouping, and the corresponding measured property data may be uploaded to the cloud where further ML based processing may be performed to identify particle lithology (or a lithology of each identified group), to determine a grain size class and distribution, extract texture features, and/or generate a consolidated listing or summary of the particle and group properties (as shown collectively at 220).

With continued reference to FIG. 2, the obtained digital image(s) may be processed with a segmenting algorithm at 212 to obtain segmented images in which individual ones of the image cuttings particles are identified (e.g., highlighted, outlined, or otherwise demarcated). Features and/or properties of the identified particles in the segmented images may be extracted at 214. The features and/or property extraction may include both an AI-based extraction of AI generated features and a non-AI based extraction of particle features and/properties (e.g., such as color and shape related measurements). The segmented particles may be grouped at 216 based on the extracted features to form one or more particle groups (e.g., groups of particles having common color features and/or other features that indicate a likelihood of a common lithology). The grouping may include both machine learning (ML)-based and non-ML based processing. At 218 additional properties and measured characteristics of one or more of the groups identified at 216 may be received. For example, rig personnel (i.e., a user) may be prompted (or provided an opportunity) to input additional information such as a measured data or a written description of one or more of the groups. An intermediate cuttings description including the segmented images, the grouping, and any corresponding measured property data and/or user comments may be uploaded at 219 to the cloud resources 120 (FIG. 1) where a lithology classification may be performed using an ML-based algorithm at 222 and consolidated results may be prepared and output (e.g., saved and/or downloaded to the rig) at 224.

With further reference to FIG. 2, it will be appreciated that the disclosed embodiments are not limited to any particular image acquisition methodology at 202. In example embodiments, retrieved cuttings particles (e.g., from the shale shaker or other solids control equipment) may be prepared for imaging, for example, by washing and then drying in an oven and/or sieving or meshing the cuttings to remove excessively large and/or small particles. The cuttings particles may further be placed in a tray, e.g., having a high contrast (vivid) background color to enhance particle identification and segmentation in the acquired images. The tray of particles may be placed in front of a digital camera (e.g., located in the laboratory 80) and a high resolution digital photo taken (e.g., with visible and/or UV lighting). The image acquisition process may make use of standardized and/or calibrated lighting, color enhancement, magnification, and/or focus/resolution settings. Images may be taken when the cuttings are wet or dry, with the humidity generally being controlled for dry cuttings images. Notwithstanding the foregoing, the disclosed embodiments are not limited to any image acquisition methodology.

The acquired images may optionally be labeled with a lagged depth based on a known or computed lagged depth of the cuttings sample. It will be appreciated that the lagged depth may be determined from the time at which the drill cuttings sample emerges from the well or at which the cuttings are collected from the shale shaker and the time delay (the lag time) required to transport the cuttings up the borehole annulus to generate a time at which the cuttings were generated in the well (e.g., by subtracting the lag time from the time at which the cuttings arrive at the surface). This time may then be correlated with a measured depth log to determine the lagged depth.

The obtained digital image(s) may be processed with a segmenting algorithm at 212 to obtain segmented images in which a plurality of individual cuttings particles are identified (e.g., highlighted or outlined) in the segmented image(s). In example embodiments, the segmenting algorithm may employ a Mask Region-Based Convolutional Neural Network (Mask R-CNN) such as disclosed in commonly assigned U.S. Patent 12,131,526. The Mask R-CNN may be configured to identify the individual cuttings particles in the digital images and thereby generate the segmented image at 212. For example, the Mask R-CNN may produce, for example, bounding boxes and mask images. The bounding boxes may be defined as a set of x-y coordinates in an image that indicates an image region that contains an object of interest. The bounding box may include a confidence score that ranges from 0 to 1 (e.g., with greater values indicating higher confidence regarding) for each object of interest. The mask image may indicate (e.g., highlight or otherwise bound) regions of interest that have a confidence score that exceeds a threshold.

It will be appreciated that Mask R-CNN is a model architecture that falls in the supervised learning category, meaning that it commonly requires a training dataset that includes images and corresponding labels. For example, the model may be trained using images including cuttings particles of various sizes, shapes, colors, and lithology types. It will be further appreciated that the R-CNN model may be continuously retrained during a drilling operation or between drilling operations in a field. For example, segmentation errors may be identified and corrected and then used to generate labeled training images that may be used to retrain (or further train) the R-CNN.

In example embodiments, a segmented image may depict a plurality of cuttings particles on a tray. The individual particles may be identified, for example, via a particle outline or other demarcation. Moreover, each identified particle may be identified by a corresponding set of pixels in the image. Stated another way the segmented image may include a pixel by pixel segmentation in which each pixel in the image is assigned to the background or to a single individual particle (an instance).

The extracted features at 214, may include, for example, color related features, size and shape related features, as well as AI generated features extracted using an ML-based algorithm. The color related features may include, for example, average (such as mean, median, or mode) red, green, and blue intensities or distributions of or standard deviations of red, green, and blue intensities and/or an average luminance of each particle. The color related features may further include a histogram, a variance, a skewness, and/or a kurtosis of the red, green, and blue intensities. Moreover, for infrared and/or ultraviolet images, the color related features may include average (such as mean, median, or mode) infrared or ultraviolet intensities or distributions of or standard deviations of infrared or ultraviolet intensities and/or an average infrared or ultraviolet luminance of each particle. The color related features may further include a histogram, a variance, a skewness, and/or a kurtosis of the infrared or ultraviolet intensities.

The size and shape related features may include, for example, a particle diameter, an area, a perimeter, a maximum axis, a minimum axis, a particle aspect ratio, and internal angle measurements. Moreover, the geometry related features may further include spatial relationships of the pixels grouped in each particle to extract particle circularity, solidity, elongation, roundness, and/or convex hull area. Individual particles may further be classified, for example, as being a plate, a fiber, circular or oval particulate, sharp angled particulate, etc. as well as being classified in one of various size bins (e.g., based on the diameter, cross sectional area, and/or perimeter of the particle).

The extracted features may further include texture related features that quantify various spatial relationships and/or directional changes in pixel color and/or brightness in each particle. Extracted texture related features may include, for example, edge detection, pixel to pixel contrast, correlation, and/or entropy. In addition, in certain embodiments, texture related features may be extracted with techniques such as image texture filters (e.g., Gabor filters, and so forth), an autoencoder, or other deep learning based techniques. In example embodiments, the AI generated features may include AI generated texture related features that are extracted using deep learning (AI or ML) based techniques.

The particles may be grouped at 216, for example, according to a "location" of individual particles in a multi-dimensional space of the extracted color, size, shape, and/or AI generated features. For example, as described above, a set of particle features may be extracted and/or computed for each of the selected cuttings particles. This set of particle features may include substantially any suitable number of features and sometimes may include a large number of features, for example, including up to 16 features (e.g., up to 32, 48, 64, or more features). In example embodiments, the particles may be clustered in a multidimensional hyperspace of the extracted and/or computed features. While the disclosed embodiments are not limited in this regard, the clustering or grouping may include a nearest neighbor regression based on the distance (e.g., the Euclidean distance) between individual cuttings particles in the multidimensional hyperspace. It will be appreciated that there is no training step when using a nearest neighbor regression method. In such embodiments, a local region or a group may be defined by the number K-nearest neighbor reference cuttings, where K may be substantially any number of nearest neighbors (such as 5, 10, 20, or 50). In other embodiments, the grouping at 216 may include (or further include) the use of a ML algorithm or a retrained ML (or AI) algorithm.

With continued reference to FIG. 2, rig personnel may be prompted (or provided opportunity) to input particle property and/or measurement data as well as well as other comments or description. It will be appreciated that the input is optional. For example, the embedded unit 110 may provide a listing of the particle groups to the user with corresponding images of representative particles in each group along with a prompt to enter property and/or measurement data. The property and/or measurement data may include substantially any suitable information, for example, including a hardness measurement, a porosity measurement, a UV fluorescence measurement, a grain size measurement, an XRF elemental analysis (or summary thereof), DRIFTS mineralogy measurements, and/or the results of an acid test in which the particles may be subject to an acid (e.g., hydrochloric or sulfuric acid) and the reactivity of the particle observed. It will be appreciated that the measured properties may be quantitative and/or qualitative depending on the nature of the measurements. In example embodiments, the input particle property and/or measurement data may further include a user input lithology label, such as sandstone, limestone, etc. such that each group or cluster of particles receives a corresponding lithology label.

The cloud resources are configured to provide a lithology classification for each of the particles (or each of the particle groupings) using an ML-based algorithm at 222. In example embodiments, the segmented images and the extracted features may be processed with the ML-based algorithm to classify the identified cuttings particles and/or particle groups with corresponding lithology types, for example, as described in commonly assigned U.S. Patent Application Publication 20230220770A1. In example embodiments, a convolutional neural network (CNN) or a transformer model may be trained on datasets of drill cutting sample images collected from lithologies around the world or on images obtained from sample images collected from lithologies obtained locally (e.g., in the local basin or oilfield). The training images may be manually annotated at both the pixel-level (assigning a rock type label to each image pixel) and/or at the rock or particle level (assigning a rock type label to each rock or particle in the image).

It will be appreciated that the lithology of a rock or cuttings particle is a description of its physical characteristics visible at an outcropping, in hand or core samples, or with low magnification microscopy. Lithology may refer to either a detailed description of these physical characteristics, or a summary of the gross physical character of a rock. In a second sense, the lithology of a rock (or cuttings particle) refers to a type of rock or a gross (or macro) identification or classification of the rock. The term lithology is generally used herein in this second sense. Example lithologies or lithology types in this second sense include sandstone, limestone, slate, shale, basalt, coal, anhydrite, dolomite, gypsum, clay, chert, granite, and the like. As such, the extracted features, such as the color and/or texture features represent physical characteristics of a lithology that may be measured and quantified from a digital image and evaluated to determine the lithology of a formation (in the second sense). For example, the color and/or texture features extracted from selected particles in the segmented image may be evaluated using a trained model (such as a trained CNN or transformer model) to classify the lithology type(s) of those selected particles.

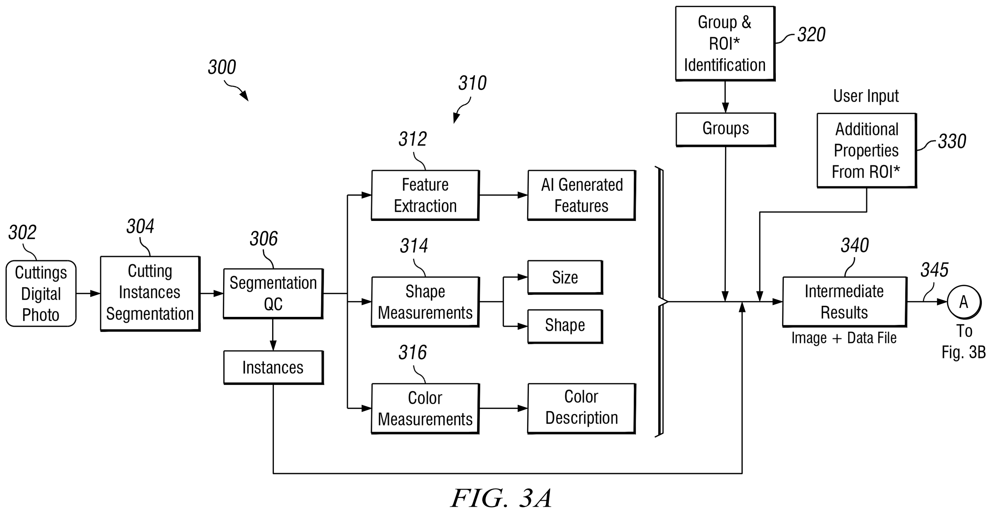

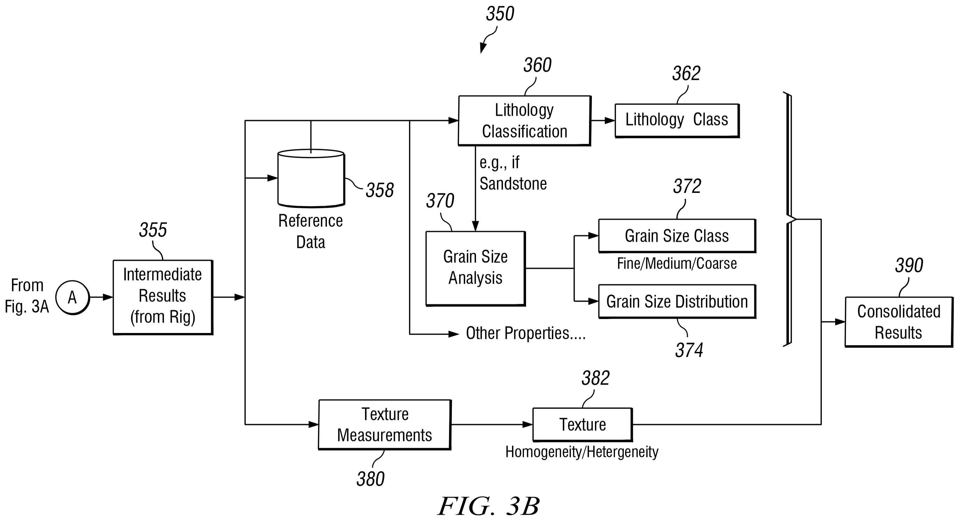

FIGS. 3A and 3B (collectively FIG. 3) depict a flow chart of another example method 300 for generating a cuttings description during a drilling operation. Method 300 is similar to method 200 in that it includes obtaining or taking a digital image of drill cuttings particles at 302, processing the obtained images at the rig site (as shown collectively at 310 in FIG. 3A) to determine a particle grouping and to prompt rig personnel to acquire and/or input measured physical property data for one of more of the particle groups (e.g., each of the particle groups). The digital images, the grouping, and the corresponding measured property data may be uploaded to the cloud where further ML-based processing may be performed to identify particle lithology (or a lithology of each identified group), to determine a grain size class and distribution, extract texture features, and generate a consolidated listing or summary of the particle and group properties (as shown collectively at 350 in FIG. 3B).

In FIG. 3A, a digital image of the drill cuttings particles is acquired at 302, for example, as described above with respect to FIG. 2. The image may be segmented at 304 to obtain a segmented image including identified (e.g., highlighted, outlined, or otherwise demarcated) cuttings particles as also described above with respect to FIG. 2. In example embodiments, a segmentation quality control routine may be performed at 306 prior to outputting the segmented image. The segmentation quality control routine may include, for example, comparisons of the segmented particle geometry (e.g., size and shape) with predefined physical constraints and statistical representations of typical cuttings instances. In other words, the segmented instances may be compared with various upper and lower geometry thresholds that enable poor segmentation results to be identified (e.g., a single instance including multiple particles or a single particle being split into multiple instances). In example embodiments, the quality control routine may further prompt user input and/or validation when necessary. Such an approach may improve the overall segmentation quality and further ensure that the grouping is both robust and user validated.

The segmented images (including the identified cuttings particles) may be further processed at 312, 314, and 316 to extract and/or measure various features of the identified particles and in so doing output a set of alphanumeric data as intermediate processing results of the image analysis. For example, as described above with respect to FIG. 2, AI generated features may be extracted at 312 using a ML algorithm, size and shape related features may be extracted and/or measured at 314, and color related features may be extracted and/or measured at 316. It will be appreciated that the color, size, and shape related features do not generally require a ML or AI algorithm.

The extracted features may be further evaluated at 320 to group the particles into one or more groups having common or similar features. As described above, the particles may be grouped at 320 according to a "location" of individual particles in a multi-dimensional space of the extracted color, size, shape, and AI generated features. It will be appreciated that in some embodiments, certain ones of the extracted features may be prioritized or weighted more heavily than other ones of the extracted features. For example only, color features may be prioritized in some embodiments.

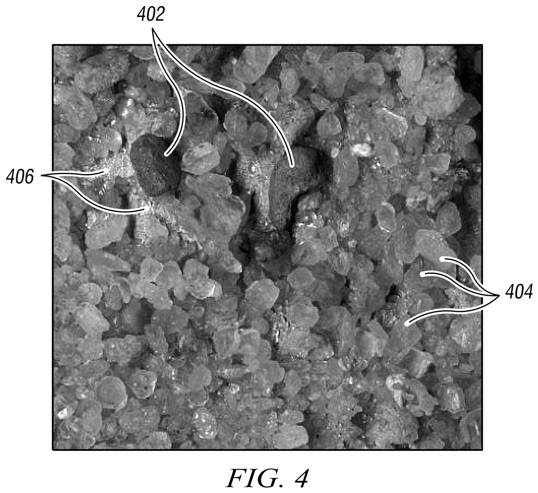

FIG. 4 depicts an example digital image of cuttings particles including first and second groups corresponding to first and second lithologies. In the example image depicted, the particles were grouped using the features extracted at 312, 314, and 316. Example first and second groups are notated at 402 and 404. The tray background is notated at 406. While FIG. 4 depicts a black and white (grey scale) image, it will be appreciated that the disclosed embodiments generally employ high resolution color images of the cuttings particles.

With continued reference to FIG. 3A, particle property and/or measurement data may be input at 330. In example embodiments, the software built atop the AI layer may interact with the user to obtain additional information related to the cuttings particles to label the data and particle groups and thereby increase the quality of the analysis. For example, rig personnel may be prompted to optionally input the data as well as well as other comments or a written particle description. As described above, the embedded unit 110 may provide a listing of the particle groups to the user with corresponding images of representative particles in each group along with a prompt to enter the data and/or description. Example inputs are described above with respect to FIG. 2.

The segmented images, the extracted features, the grouping, and the user input information may be combined together as intermediate results at 340. These intermediate results may then be uploaded to the cloud or other central processing facility for further processing as depicted at 345. It will be appreciated that the above described intermediate results may be transmitted to the offsite location at substantially any suitable time or measured depth interval. In example embodiments, the intermediate results may be uploaded one segmented image at a time (e.g., segmented image by segmented image). In other embodiments, the intermediate results may be uploaded less frequently, for example, in batches of multiple segmented images or at a predetermined time or depth interval including multiple segmented images (and corresponding data). The disclosed embodiments are expressly not limited in this regard.

It will be further appreciated that the intermediate results do not necessarily include user input information for each segmented image. Nor do they necessarily include user input information for each of the groups identified in the segmented images. Moreover, in some embodiments, it may be advantageous to add more extensive input measurement data when a new or previously unidentified group of cuttings particles is identified (e.g., representative of a new strata or subterranean layer.

In FIG. 3B, the uploaded intermediate results are received at 355. The user input data 330 may be added to a reference database 358. As such, the user inputs (the input data and comments) may be regarded as labels and may be merged with the AI generated data. An AI-based lithology classification is conducted at 360 to obtain a labeled image in which selected ones of the segmented cuttings particles are labeled with a corresponding lithology type 362. The classification may be performed, for example, using a trained CNN or transformer model as described above with respect to FIG. 2. The segmented image may be further evaluated (along with the lithology classification) to perform a grain size evaluation of selected cuttings particles at 370 (e.g., particles having a particular lithology label such as sandstone) and may be based on the extracted color, size, shape, and AI-generated features. The grain size evaluation 370 may output, for example, a grain size class at 372 (such as fine, medium, or coarse-grained) and a grain size distribution class at 374 (such as narrow, medium, or broadly distributed).

With continued reference to FIG. 3B, the received intermediate results may be further evaluated to make texture measurements at on (or extracted texture related features from) the particles in the segmented image at 380. The texture measurements may be intended to quantify various spatial relationships and/or directional changes in pixel color and/or brightness in each particle. Extracted texture related features may include, for example, edge detection, pixel to pixel contrast, correlation, and/or entropy. In addition, in certain embodiments, texture related features may be extracted with techniques such as image texture filters (e.g., Gabor filters, and so forth), an autoencoder, or other deep learning based techniques. Moreover, directional changes may be evaluated, for example, for symmetry and used to generate spectra that may be further compared with reference spectra to assign a texture classification to each particle, such as homogeneous, heterogeneous, grainy, laminate, etc. at 382. Although not depicted in FIG. 3B, the texture features may also be considered in the grain size analysis at 370 to characterize the grain size and/or grain size distribution of grains in the cuttings particles. The extracted features and user input may be optionally further evaluated in the cloud to perform additional feature extraction and/or classifications, for example, related to the geometrical, mechanical, and geological properties of the cuttings particles, such as cuttings shape, hardness, and porosity.

A consolidated summary of the results may be generated at 390 and may include, for example, the segmented image, the features extracted at 312, 314, and 316 as well as the texture related features extracted at 390, the lithology classification, and the grain size and grain size distribution classifications. The consolidated results summary may be saved to memory and/or electronically transferred back to the rig site at which the digital image was acquired.

It will be appreciated that the disclosed system and computerized method for evaluating drill cuttings images may confer several advantages. For example, each embedded unit (and corresponding rig site) performs its own analysis in which the heavy (a processor intensive) computations are not all performed at the same place. This tends to reduce accumulation of data at a single remote location (the cloud) and thereby tends to reduce lag time and promote efficient and reliable service (in terms of service availability). Moreover, each embedded unit is autonomous and delivers pre-analyze data in addition to the segmented image. Such autonomy advantageously promotes adaptability which in turn enables local processing to be tailored to local lithologies and cuttings properties (e.g., via retraining or supplemental training of the local AI algorithms using local knowledge and corresponding labeling).

It will be understood that the present disclosure includes numerous embodiments. These embodiments include, but are not limited to, the following embodiments.

In a first embodiment, a computerized method for evaluating a drill cuttings lithology comprises acquiring a digital image of drill cuttings at a rig site; segmenting the acquired digital image at the rig site to demarcate individual particles in the digital image; extracting features from selected ones of the demarcated particles in the segmented image at the rig site; assigning the demarcated particles to at least one group at the rig site based on the extracted features; prompting a rig site user to input measurement data, a label, or a description of the at least one group; electronically transferring the segmented image, the extracted features, the assigned grouping, and the user input to a cloud computing resource; performing a lithological classification of selected ones of the demarcated particles using the cloud computing resource, the lithological classification based on the extracted features and the user input; and generating consolidated summary results of the lithological classification of the segmented image.

A second embodiment may include the first embodiment, wherein the acquiring the digital image comprises drilling the subterranean wellbore; collecting the drill cuttings particles from circulating drilling fluid; preparing the drill cuttings particles for imaging; and taking the digital image of the prepared drill cuttings particles at the rig site.

A third embodiment may include any one of the first through second embodiments, wherein the segmented image comprises a pixel by pixel segmentation in which each pixel in the image is assigned to a background or to a single one of the demarcated particles.

A fourth embodiment may include any one of the first through third embodiments, wherein the extracted properties comprise color related features, size and shape related features, and AI generated features.

A fifth embodiment may include any one of the first through fourth embodiments, wherein the assigning the demarcated particles to at least one group further comprises clustering the demarcated particles in a multidimensional hyperspace of selected ones of the extracted features.

A sixth embodiment may include any one of the first through fifth embodiments, wherein the user input comprises at least one of a hardness measurement, a fluorescence measurement, a measurement of chemical reactivity with an acid, and a lithological description.

A seventh embodiment may include any one of the first through sixth embodiments, wherein the user input is saved in a reference database in the cloud computing resource and the lithological classification is based on the extracted features and the user input in the reference database.

An eighth embodiment may include any one of the first through seventh embodiments, wherein the segmented image is further processed using the cloud resources to extract texture related features, the texture related features quantifying spatial relationships or directional changes in pixel color or brightness in each of the demarcated particles.

A ninth embodiment may include any one of the first through eighth embodiments, further comprising performing a grain size evaluation of selected ones of the demarcated particles using the cloud resources, the grain size evaluation based on the lithological classification and the extracted features, the grain size evaluate outputting a grain size classification and a grain size distribution classification.

A tenth embodiment may include any one of the first through ninth embodiments, wherein the consolidated summary results further comprise texture related features quantifying spatial relationships or directional changes in pixel color or brightness in each of the demarcated particles extracted by the cloud resources and a grain size classification and a grain size distribution classification determined by the cloud resources.

In an eleventh embodiment as system for evaluating a drill cuttings lithology comprises a digital camera located at a rig site, the digital camera configured to acquire a digital image of drill cuttings; a first processor located at the rig site, the first processor configured to segment the acquired digital image to demarcate individual particles and thereby generate a segmented image; extract features from selected ones of the demarcated particles in the segmented image; assign the demarcated particles to at least one group based on the extracted features; prompt a rig site user to input measurement data, a label, or a description of the demarcated particles in the at least one group; and electronically transfer the segmented image, the extracted features, the assigned grouping, and the user input to a cloud location; and a second processor located at the cloud location, the second processor configured to: receive the electronic transfer from the first processor; perform a lithological classification of selected ones of the demarcated particles, the lithological classification based on the extracted features and the user input; and generate consolidated summary results of the lithological classification of the segmented image.

A twelfth embodiment may include the eleventh embodiment, wherein the first processor is configured to automatically segment the acquired digital image, extract the features, assign the demarcated particles to at least one group, prompt the rig site user, and electronically transfer the segmented image, the extracted features, the assigned grouping, and the user input to the cloud location; and the second processor is configured to automatically receive the electronic transfer, perform the lithological classification, and generate the consolidated summary results.

A thirteenth embodiment may include any one of the eleventh through twelfth embodiments, wherein the first processor is configured to segmented the acquired digital image by performing a pixel by pixel segmentation in which each pixel in the image is assigned to a background or to a single one of the demarcated particles.

A fourteenth embodiment may include any one of the eleventh through thirteenth embodiments, wherein the first processor is configured to the extracted features comprising color related features, size and shape related features, and AI generated features.

A fifteenth embodiment may include any one of the eleventh through fourteenth embodiments, wherein the first processor is configured to assign the demarcated particles by clustering the demarcated particles in a multidimensional hyperspace of selected ones of the extracted features.

A sixteenth embodiment may include any one of the eleventh through fifteenth embodiments, wherein the first processor is configured to prompt the user to at least one of a hardness measurement, a fluorescence measurement, a measurement of chemical reactivity with an acid, and a lithological description.

A seventeenth embodiment may include any one of the eleventh through sixteenth embodiments, wherein the second processor is further configured to save the user input to a reference database in the cloud location and perform the lithological classification based on the extracted features and the user input in the reference database.

An eighteenth embodiment may include any one of the eleventh through seventeenth embodiments, wherein the second processor is further configured to process the segmented image to extract texture related features, the texture related features quantifying spatial relationships or directional changes in pixel color or brightness in each of the demarcated particles.

A nineteenth embodiment may include any one of the eleventh through eighteenth embodiments, wherein the second processor is further configured perform a grain size evaluation of selected ones of the demarcated particles, the grain size evaluation based on the lithological classification and the extracted features, the grain size evaluate outputting a grain size classification and a grain size distribution classification.

A twentieth embodiment may include any one of the eleventh through nineteenth embodiments, wherein the consolidated summary results further comprise texture related features quantifying spatial relationships or directional changes in pixel color or brightness in each of the demarcated particles extracted by the cloud resources and a grain size classification and a grain size distribution classification determined by the cloud resources.

Although automated rig site lithology data extraction has been described in detail, it should be understood that various changes, substitutions and alternations can be made herein without departing from the spirit and scope of the disclosure as defined by the appended claims.

## Claims

1. A computerized method for evaluating a drill cuttings lithology, the method comprising:
acquiring a digital image of drill cuttings at a rig site;
segmenting the acquired digital image at the rig site to demarcate individual particles in the digital image;
extracting features from selected ones of the demarcated particles in the segmented image at the rig site;
assigning the demarcated particles to at least one group at the rig site based on the extracted features;
prompting a rig site user to input measurement data, a label, or a description of the at least one group;
electronically transferring the segmented image, the extracted features, the assigned grouping, and the user input to a cloud computing resource;
performing a lithological classification of selected ones of the demarcated particles using the cloud computing resource, the lithological classification based on the extracted features and the user input; and
generating consolidated summary results of the lithological classification of the segmented image.

2. The method of claim 1, wherein the acquiring the digital image comprises:
drilling the subterranean wellbore;
collecting the drill cuttings particles from circulating drilling fluid;
preparing the drill cuttings particles for imaging; and
taking the digital image of the prepared drill cuttings particles at the rig site.

3. The method of claim 1 or 2, wherein the segmented image comprises a pixel by pixel segmentation in which each pixel in the image is assigned to a background or to a single one of the demarcated particles.

4. The method of claim 1, 2 or 3, wherein the extracted properties comprise color related features, size and shape related features, and AI generated features.

5. The method of any one of the preceding claims, wherein the assigning the demarcated particles to at least one group further comprises clustering the demarcated particles in a multidimensional hyperspace of selected ones of the extracted features.

6. The method of any one of the preceding claims, wherein the user input comprises at least one of a hardness measurement, a fluorescence measurement, a measurement of chemical reactivity with an acid, and a lithological description.

7. The method of any one of the preceding claims, wherein the user input is saved in a reference database in the cloud computing resource and the lithological classification is based on the extracted features and the user input in the reference database.

8. The method of any one of the preceding claims, wherein the segmented image is further processed using the cloud resources to extract texture related features, the texture related features quantifying spatial relationships or directional changes in pixel color or brightness in each of the demarcated particles.

9. The method of any one of the preceding claims, further comprising performing a grain size evaluation of selected ones of the demarcated particles using the cloud resources, the grain size evaluation based on the lithological classification and the extracted features, the grain size evaluate outputting a grain size classification and a grain size distribution classification.

10. The method of any one of the preceding claims, wherein the consolidated summary results further comprise texture related features quantifying spatial relationships or directional changes in pixel color or brightness in each of the demarcated particles extracted by the cloud resources and a grain size classification and a grain size distribution classification determined by the cloud resources.

11. A system for evaluating a drill cuttings lithology, the system comprising:
a digital camera located at a rig site, the digital camera configured to acquire a digital image of drill cuttings;
a first processor located at the rig site, the first processor configured to segment the acquired digital image to demarcate individual particles and thereby generate a segmented image; extract features from selected ones of the demarcated particles in the segmented image; assign the demarcated particles to at least one group based on the extracted features; prompt a rig site user to input measurement data, a label, or a description of the demarcated particles in the at least one group; and electronically transfer the segmented image, the extracted features, the assigned grouping, and the user input to a cloud location; and
a second processor located at the cloud location, the second processor configured to: receive the electronic transfer from the first processor; perform a lithological classification of selected ones of the demarcated particles, the lithological classification based on the extracted features and the user input; and generate consolidated summary results of the lithological classification of the segmented image.

12. The system of claim 11, wherein:
the first processor is configured to automatically segment the acquired digital image, extract the features, assign the demarcated particles to at least one group, prompt the rig site user, and electronically transfer the segmented image, the extracted features, the assigned grouping, and the user input to the cloud location; and
the second processor is configured to automatically receive the electronic transfer, perform the lithological classification, and generate the consolidated summary results.

13. The system of claim 11 or 12, wherein the first processor is configured to segmented the acquired digital image by performing a pixel by pixel segmentation in which each pixel in the image is assigned to a background or to a single one of the demarcated particles and/or wherein the first processor is configured to the extracted features comprising color related features, size and shape related features, and AI generated features and/or wherein the first processor is configured to assign the demarcated particles by clustering the demarcated particles in a multidimensional hyperspace of selected ones of the extracted features and/or wherein the first processor is configured to prompt the user to at least one of a hardness measurement, a fluorescence measurement, a measurement of chemical reactivity with an acid, and a lithological description.

14. The system of claim 11, 12 or 13, wherein the second processor is further configured to save the user input to a reference database in the cloud location and perform the lithological classification based on the extracted features and the user input in the reference database and/or wherein the second processor is further configured to process the segmented image to extract texture related features, the texture related features quantifying spatial relationships or directional changes in pixel color or brightness in each of the demarcated particles and/or wherein the second processor is further configured perform a grain size evaluation of selected ones of the demarcated particles, the grain size evaluation based on the lithological classification and the extracted features, the grain size evaluate outputting a grain size classification and a grain size distribution classification.

15. The system of any one of the claims 11 - 14, wherein the consolidated summary results further comprise texture related features quantifying spatial relationships or directional changes in pixel color or brightness in each of the demarcated particles extracted by the cloud resources and a grain size classification and a grain size distribution classification determined by the cloud resources.
